Europäisches Patentamt

European Patent Office

Office européen des brevets

(11) Numéro de publication: **0 365 390**
**A1**

## (12) DEMANDE DE BREVET EUROPEEN

(21) Numéro de dépôt: 89402758.0

(22) Date de dépôt: 06.10.89

(51) Int. Cl.⁵: **C12P 19/06 , C12P 19/04 , E21B 43/25 , //(C12P19/06, C12R1:64)**

(30) Priorité: 19.10.88 FR 8813732

(43) Date de publication de la demande:
25.04.90 Bulletin 90/17

(84) Etats contractants désignés:
AT BE CH DE ES FR GB GR IT LI LU NL SE

(71) Demandeur: **RHONE-POULENC CHIMIE**
**25, quai Paul Doumer**
**F-92408 Courbevoie Cédex(FR)**

(72) Inventeur: **Nicolas, Olivier**
**15, rue Eloi Ricard**
**F-79500 Melle(FR)**

(74) Mandataire: **Tavernier, Colette et al**
**RHONE-POULENC CHIMIE Service Brevets**
**Chimie 25, Quai Paul Doumer**
**F-92408 Courbevoie Cédex(FR)**

(54) **Procédé de production de polysaccharides par fermentation d'une source carbonée à l'aide de microorganismes.**

(57) Procédé de production de polysaccharides par fermentation d'un milieu nutritif aqueux, comprenant une source carbonée au moyen de microorganismes.

Le procédé est caractérisé par l'emploi d'une source d'azote complexe.

Les polysaccharides ainsi préparés peuvent être notamment utilisés dans le domaine de la récupération assistée du pétrole.

EP 0 365 390 A1

# PROCEDE DE PRODUCTION DE POLYSACCHARIDES PAR FERMENTATION D'UNE SOURCE CARBONEE A L'AIDE DE MICROORGANISMES

La présente invention concerne un procédé de production de polysaccharides par fermentation d'une source carbonée à l'aide de microorganismes. Elle concerne encore plus particulièrement un procédé de fermentation permettant d'obtenir des solutions aqueuses du gels de polysaccharides présentant une filtrabilité améliorée, ainsi que l'application à la récupération assistée du pétrole dédits polysaccharides.

Les polysaccharides ou biopolymères obtenus par fermentation d'une source carbonée sous l'action de bactéries du genre Xanthomonas ou Arthrobacter, de champignons appartenant au genre Sclerotium ou d'autres microorganismes du même genre ont trouvé de nombreuses applications industrielles en raison de leurs propriétés épaississantes et viscosifiantes.

Les hétéropolysaccharides de type Gomme Xanthane ont notamment trouvé une application intéressante dans les procédés de récupération secondaire et tertiaire du pétrole. Dans cette application, on utilise des solutions aqueuses diluées à une concentration d'environ 300 à 3000 ppm pour déplacer l'huile des réservoirs partiellement épuisés. La Gomme Xanthane se caractérise en effet par une haute viscosité à basse concentration, une grande insensibilité à la salinité et à la nature des sels et une grande stabilité aux contraintes mécaniques. Toutefois, les solutions préparées à partir des qualités industrielles soit à partir du moût de fermentation, soit par dilution de la poudre précipitée et séparée à partir du moût, présentent l'inconvénient majeur de colmater rapidement les pores des formations rocheuses dans lesquelles elles sont injectées, provoquant ainsi des augmentations de pression indésirables et empêchant rapidement toute récupération supplémentaire d'huile. On sait que les origines de ce colmatage sont dues à la présence d'une part de particules insolubles telles que débris cellulaires et bactéries non viables provenant de la fermentation, d'autre part d'un certain nombre d'aggrégats translucides ou microgels notamment si la solution est préparée avec du biopolymère qui a été précipité à partir du moût de fermentation.

Il a été constaté que des différences notables peuvent être observées dans le comportement rhéologique des solutions aqueuses de gomme Xanthane qui dépendent de la composition du milieu de fermentation et en particulier de la source d'azote utilisée. On sait que l'emploi de solubles de distilleries tel que décrit dans les brevets américains US 3 000 790, US 3 020 206, US 3 328 262, US 3 495 719, apporte une quantité importante d'insolubles dans le bouillon de fermentation. Pour remédier à ce problème, selon d'autres procédés connus, on utilise dans le milieu de fermentation final une source d'azote minéral et/ou une source d'azote organique en quantité mineure : voir par exemple US 3 391 060, US 3 433 708 et le brevet français N° 2 414 555.

Bien que la filtrabilité soit améliorée, ces milieux ne donnent pas entière satisfaction du point de vue productivité et/ou filtrabilité. Différentes sources d'azote organique utilisables sont citées dans ces documents mais il n'apparait pas que le choix d'un composé parmi les composés connus conduise à une efficacité particulière. Par ailleurs, l'emploi d'azote minéral rend le milieu fragile à la contamination et peut conduire en fabrication industrielle à des fermentations secondaires si le milieu n'est pas parfaitement stérile.

Des milieux de fermentation modifiés pour la préparation de polysaccharides utilisables dans le domaine de la récupération assistée du pétrole ont encore été proposés dans les brevets américains US 4 119 546, US 4 296 203, US 4 377 637 et dans la demande de brevet européen 0066961.

La présente invention a pour but de fournir un procédé de préparation de polysaccharides du type gomme Xanthane avec une productivité élevée, conduisant à des sols aqueux ayant de bonnes propriétés de viscosité et de filtrabilité. Un but plus particulier de l'invention est l'obtention de bouillons de fermentation contenant du polysaccharide produit par Xanthomonas, particulièrement aptes à êtres utilisés pour le déplacement du pétrole de réservoirs particulièrement épuisés.

la présente invention concerne donc un procédé de production de polysaccharides par fermentation au moyen de microorganismes d'un milieu nutritif aqueux contenant de 10 à120 g/l d'une source carbonée ainsi qu'une source d'azote, caractérisé en ce que la source d'azote contient de la caséine ou un dérivé de caséine en mélange avec une autre source d'azote organique et/ou minérale.

Le dérivé de caséine peut être choisi parmi les sels de caséine comme le caséinate de sodium, la caséinate de potassium, le caséinate d'ammonium, le caséinate de calcium, les hydrolysats de caséine.

Les sels de caséine peuvent être obtenus par neutralisation au sein du milieu de fermentation de la caséine. La caséine ou ses dérivés sont utilisés avantageusement selon des concentrations, exprimées en équivalent gramme d'azote élémentaire par litre du milieu de fermentation, comprises entre 0,007 et 0,5 g/l et de préférence entre 0,014 et 0,35 g/l.

Le milieu de fermentation contient en plus de la caséine une autre source d'azote organique et/ou minérale. Parmi les sources azotées organiques, on peut citer les peptones, la gélatine, l'extrait de levure, l'extrait soluble de maïs, la farine de soja.

Parmi les sources azotées minérales, on peut citer le nitrate d'ammonium, le chlorure d'ammonium, les carbonates mono ou diammonique, les sulfates mono ou diammonique, les phosphates mono ou diammoniques et les nitrates alcalins.

Comme complément à la caséine ou à ses dérivés, on donne la préférence aux sources d'azote organique. On préfère notamment utiliser l'extrait soluble de maïs (CSL) car il est parmi les sources organiques d'azote celle qui procure les meilleurs rendements. Pour augmenter la productivité, les sources d'azote organique peuvent être éventuellement enrichies avec une petite quantité d'une source d'azote minéral.

La quantité totale d'azote exprimée en équivalent grammes d'azote élémentaire par litre du milieu de fermentation est avantageusement comprise entre 0,2 et 0,6 g/l et de préférence comprise entre 0,3 et 0,4 g/l.

La source carbonée peut être un sucre. De préférence on utilisera le glucose, le saccharose, le fructose, le maltose, le lactose, le galactose, avec une concentration favorablement comprise entre 10 et 40 g/l.

Le glucose et le saccharose sont plus particulièrement préférés.

Outre la source carbonée et la source d'azote, le milieu de fermentation contient de manière connue en soi des traces d'éléments et facteur de croissance essentiels.

Le milieu de fermentation peut contenir des ions magnésium apportés par exemple par le sulfate de magnésium, l'acétate de magnésium, le chlorure de magnésium ou le nitrate de magnésium. La concentration en magnésium élémentaire est de préférence comprise entre 0,001 et 0,05 g/l.

Le milieu de fermentation peut aussi contenir une source de phosphore qui peut être mise dès le départ ou qui peut avantageusement être introduite lors de la régulation du pH à l'aide par exemple de phosphate de potassium, de sodium ou d'ammonium. La concentration en phosphore élémentaire est de préférence comprise entre 0,05 et 1,3 g/l. Le pH sera avantageusement maintenu entre 6 et 7,5.

Un agent antimousse peut être introduit également dans le milieu de fermentation.

Le microorganisme agent de la fermentation est choisi parmi les bactéries qui fermentent les hydrates de carbone telles que celles décrites dans BERGEY'S MANUAL OF DETERMINATIVE BACTERIOLOGY (8e édition - 1974 - Williams N. Wilkins C° Baltimore) et par exemple Xanthomonas begoniae, Xanthomonas campestris, Xanthomonas carotae, Xanthomonas hederae, Xanthomonas incanae, Xanthomonas malvacearum, Xanthomonas papaveri cola, Xanthomonas phaseoli, Xanthomonas pisi, Xanthomonas vasculorum, Xanthomonas vesicatoria, Xanthomonas vitians, Xanthomonas pelargonii ; les bactéries du genre Arthrobacter et plus particulièrement les espèces Arthrobacter stabilis, Arthrobacter viscosus ; un genre Erwinia ; du genre Azobacter et plus particulièrement l'espèce Azobacter indicus ; du genre Agrobacterium et plus particulièrement les espèces Agrobacterium radiobacter, Agrobacterium rhizogenes, Agrobacterium tumefaciens. Peuvent aussi être utilisés les champignons appartenant au genre Sclerotium.

On préfère utiliser parmi ces microorganimes Xanthomonas campestris.

Le microorganisme est introduit dans le milieu de fermentation de manière connue en soi et par exemple à l'aide de cultures intermédiaires obtenues dans des fermenteurs de laboratoire de 20 litres qui elles-mêmes ont été obtenues à partir d'inoculum réalisé par exemple en erlenmeyer d'un litre.

La préparation des inoculums ou des cultures intermédiaires connue de tout homme de l'art est décrite par exemple dans le brevet français publié sous le n° 2 414 555.

La fermentation est ensuite réalisée pendant quelques jours par le procédé selon l'invention en une ou plusieurs étapes de croissance et de production. Le milieu de croissance et le milieu de production peuvent avoir la même composition ou une composition différente. A l'issue de la fermentation, le moût contient le polysaccharide à une concentration d'environ 15 à 50 g/litre, des débris cellulaires, les impuretés des sources carbonées ou azotées, des protéines résiduelles et des ions minéraux. A l'achèvement de la fermentation, et avant de procéder à l'isolement du polysaccharide dans le cas où on désire celui-ci sous forme de poudre, on procède de préférence à un chauffage du moût fermenté pendant 1 à 60 minutes à une température de 60 à 120°C et de préférence de 80 à 110°C. Le polymère peut être utilisé tel quel ou récupéré du moût par toute méthode connue de l'homme de l'art telle que la précipitation à l'aide d'alcool inférieur comme par exemple : le méthanol, l'éthanol, l'isopropanol, le butanol et à l'aide d'un sel minéral comme par exemple le chlorure de potassium, de sodium ou le sulfate de sodium ou le phosphate de sodium.

Le polysaccharide précipité est séparé, lavé puis séché et broyé. On obtient ainsi une poudre prête à l'emploi.

Dans le cadre de la présente invention, on peut utiliser le polysaccharide dans le moût directement ou à partir de la poudre mais il est connu que les gels obtenus par remise en suspension de la poudre sont généralement plus difficiles à filtrer que les moûts de fermentation. On peut bien entendu si on le désire ajouter un biocide ou une enzyme dans le moût de fermentation, avant traitement thermique s'il y a lieu ou à la poudre précipitée. Il peut encore être avantageux dans certains cas de procéder à une concentration du moût qui peut être réalisée par tout moyen conventionnel.

Par le présent procédé, il est possible d'obtenir avec une productivité élevée une gomme Xanthane de haute viscosité et de bonne filtrabilité.

Les bouillons entiers de fermentation et les poudres de polysaccharides peuvent être utilisés dans toutes les applications connues de la gomme xanthane, notamment les applications demandant une bonne filtrabilité, et plus particulièrement dans la récupération assistée du pétrole.

L'invention sera plus précisément décrite à l'aide des exemples suivants qui ne doivent pas être considérés comme limitatifs de l'invention.

Exemple 1 :

A partir d'une culture de Xanthomonas campestris ATCC 13951 maintenue sur gelose en tube, ou ensemence 100 ml de milieu MY-saccharose (extrait de levure 3 g ; extrait de malt 3 g ; bactopeptone 5 g ; saccharose 10 g ; eau potable q.s.p. 1 litre). La durée d'incubation à 28°C sous agitation constante est de 24 heures.

A l'aide de 100 ml de cette culture, on inocule 15 litres d'un milieu de production stérile ayant la composition suivante :

| Saccharose | 21 g/l |
| MgSO$_4$, 7 H$_2$O | 0,25 g/l |
| Caséinate de sodium | 0 à 2 g/l |
| Extrait soluble de maïs | 8,5 à 0,5 g/l |
| Huile de colza | 0,5 ml/l |
| Eau | quantité suffisante pour 1 litre |

Le pH avant inoculation est de 7,2. La fermentation se fait sous agitation et aération à la température de 28°C, le pH étant régulé à 7,2 par addition de soude.

La fermentation est arrêtée quand il n'y a plus de trace de sucre.

On opère ensuite un traitement thermique du moût puis le polysaccharide est précipité par addition d'isopropanol, lavé, séché et broyé.

La composition exacte de la source azotée et les résultats de fermentation sont donnés dans le tableau suivant.

|  | Essai 1 | Essai 2 |
|---|---|---|
| Caséinate de sodium | 0 | 2 g/l |
| Extrait de soluble de maïs | 8,5 g/l | 0,5 g/l |
| Azote total eq g/l | 0,293 g/l | 0,294 g/l |
| Durée de la fermentation | 50 h | 60 h |
| Viscosité fin de fermentation en Pa.s | 4,6 | 4,4 |
| Rendement polysaccharide/saccharose | 76 % | 59 % |

A partir de la poudre obtenue, on prépare des solutions diluées à la concentration de 0,1 % en poids en utilisant pour la dilution une solution aqueuse contenant 0,075 g/l de Ca Cl$_2$. On mesure la viscosité et la filtrabilité des solutions.

La viscosité est mesurée à l'aide d'un viscosimètre Brookfield modèle LVT - équipement UL - 6 t/mn - 25°C.

La filtrabilité est évaluée par passage de la solution à travers une membrane poreuse de diamètre de pores 12 $\mu$, sous une pression constante de 0,06 bar. On mesure le volume filtré en 30 minutes.

|  | Essai 1 | Essai 2 |
|---|---|---|
| Viscosité de la solution à 0,1 % | 21 mPas | 42 mPas |
| Volume filtré | Colmate | 446 ml |

On constate que le milieu sans caséinate donne un sol moins visqueux et qui colmate.

Exemple 2 :

Le milieu de fermentation a la composition suivante :

| Saccharose | 27 g/l |
|---|---|
| Caséinate | 1 g/l |
| Extrait soluble maïs | 2 g/l |
| KNO$_3$ | 1 g/l |
| Huile de colza | 0,5 g/l |
| MgSO$_4$ 7H$_2$O | 0,25 g/l |
| Eau | q.s.p. 1 litre |

L'azote total est de 0,35 équivalent g/l d'azote élémentaire. La durée de fermentation est de 62 heures et la viscosité finale, de 5.400 cps. Le rendement par rapport au saccharose est de 67,6 %.

La viscosité du moût est mesurée sur une solution de 0,4 g/l de polysaccharide ; elle est de 6,3 mPa.s et la viscosité d'une solution formée avec la poudre récupérée à partir du moût est de 6,9 mPa.s (Brookfield - LVT - équipement UL - 30 tours/mn - 25° C).

La filtrabilité est mesurée sur une solution à 0,04 % dans une saumure constituée de :

| NaCl | 70,5 g/l |
|---|---|
| CaCl$_2$ | 17,5 g/l |

sur une membrane présentant un diamètre de pores de 3 $\mu$ sous une pression de 10 millibars. A partir du moût, on filtre 1.978 ml en 150 mn et à partir de la poudre 495 ml en 150 minutes.

Exemple 3 :

Le milieu de fermentation a la composition suivante :

| Glucose, 1 H$_2$O | 20 g/l |
|---|---|
| Amidon | 2 g/l |
| Extrait soluble de maïs | 0,5 g/l |
| Caséinate de sodium | 1,25 ou 2,5 g/l |
| K$_2$HPo$_4$ | 7,3 g/l |
| Mg So$_4$ | 0,25 g/l |
| Huile de colza | 2,5 ml/l |
| Eau | q.s.p. 1 litre |

|  | Essai 3 | Essai 4 |
|---|---|---|
| Caséinate de sodium | 1,25 g/l | 2,5 g/l |
| Temps d'incubation et viscosité en Pa.s | 42 h 1,6 | 42 h 8,0 |
| Durée de fermentation finale et viscosité en Pa.s | 92 h 7,9 |  |
| Equivalent g d'azote total | 0,19 | 0,36 |

La viscosité du sol est mesurée comme à l'exemple 1 sur une solution à 0,1 % dans une saumure à 75 ppm de Ca Cl₂ à l'aide d'un viscosimètre Brookfield/LVT tournant à 3 tours/mn.

La filtrabilité est mesurée sur une solution à 0,1 % en poids dans une saumure constituée de CaCl₂ à 0,075 g/l sur une membrane présentant un diamètre de pores de 12 μ pendant une durée de 10 mn.

|  | Essai 3 | Essai 4 |
|---|---|---|
| Caséinate de sodium | 1,25 g/l | 2,5 g/l |
| Viscosité | 34 mPas | 90 mPas |
| Filtrabilité | 1.470 ml | 552 ml |

Exemple 4 :

Le milieu de fermentation a la composition suivante :

| Caséinate de sodium | 0 à 0,5 g/l |
|---|---|
| NH₄NO₃ | 1,2 g/l |
| K₂HPo₄ | 7,3 g/l |
| MgSo₄, 7H₂O | 0,25 g/l |
| Huile de colza | 2,5 ml/l |
| Glucose, 1H₂O | 20 g/l |
| Amidon | 2 g/l |
| Eau | quantité suffisante pour 1 litre. |

| Caséinate de sodium | 0 | 0,125 g/l | 0,5 g/l |
|---|---|---|---|
| Azote total meq g/l | 0,420 g/l | 0,437 g/l | 0,489 g/l |
| Viscosité à 24 h. en mPa.s | 200 | 400 | 1.000 |
| Durée de la fermentation | 50 h | 45 h | 42 h |
| Viscosité à fin de la fermentation en Pa.s | 5,4 | 6,0 | 5,0 |
| Rendement polysacch./Glucose | 72,5 % | 71 % | 64,5 % |

La viscosité de la poudre de polysaccharide après extraction et séchage est mesurée sur une solution contenant 0,1 % en poids de polysaccharide dans l'eau distillée à d'aide d'un viscosimètre Brookfield (LVT - équipement UL - 3 tours/mn - 25° C).

La filtrabilité de la poudre de polysaccharide est mesurée sur une solution à 0,04 % en poids de polysaccharide dans une saumure de composition suivante :

6

| NaCl | 160 g/l |
|---|---|
| CaCl$_2$, 2H$_2$O | 16,5 g/l |
| MgCl$_2$, 6H$_2$O | 7,5 g/l |

Cette solution est passée à travers un filtre poreux de diamètre de pores 3 $\mu$ sous une pression constante de 0,06 bar. On mesure le volume filtré en 30 minutes.

| Caséinate de sodium | 0 | 0,125 g/l | 0,5 g/l |
|---|---|---|---|
| Viscosité de la solution à 0,1 % | 48,2mPa.s | 86mPa.s | 114mPa.s |
| Filtrabilité | 61 ml | 87 ml | 82 ml |

## Revendications

1. Procédé de production de polysaccharides pour la préparation de solutions aqueuses ayant de bonnes propriétés de viscosité et de filtrabilité par fermentation au moyen de microorganismes d'un milieu nutritif aqueux comprenant de 10 à 120 g/l d'une source carbonée ainsi qu'une source azotée, caractérisé en ce que la source azotée contient de la caséine ou un dérivé de caséine en mélange avec une autre source d'azote organique et/ou minérale.

2. Procédé selon la revendication 1 caractérisé en ce que le dérivé de la caséine est choisi dans le groupe des sels de caséine et des hydrolysats de caséine.

3. Procédé selon la revendication 1 caractérisé en ce que l'autre source d'azote organique est choisie parmi l'extrait soluble de maïs, les peptones, la farine de soja, l'extrait de levure et la gélatine.

4. Procédé selon la revendication 1 caractérisé en ce que l'autre source d'azote est choisie parmi les sels minéraux d'ammonium et les nitrates alcalins.

5. Procédé selon l'une des revendications 1 ou 2 caractérisé en ce que la source azotée est composée de caséinate de sodium et d'extrait soluble de maïs.

6. Procédé selon l'une des revendications 1 à 5 caractérisé en ce que la quantité de caséine ou de dérivé de caséine exprimée en équivalent gramme d'azote élémentaire par litre du milieu de fermentation est comprise entre 0,007 et 0,5 g/l et de préférence entre 0,014 et 0,35 g/l.

7. Procédé selon l'une des revendications 1 à 6 caractérisé en ce que la quantité totale d'azote exprimée en équivalent gramme d'azote élémentaire par litre du milieu de fermentation est comprise entre 0,2 et 0,6 g/l et de préférence comprise entre 0,3 et 0,4 g/l.

8. Procédé selon l'une des revendications précédentes caractérisé en ce que la source carbonée est un sucre.

9. Procédé selon la revendication 8 caractérisé en ce que le sucre est le glucose, le saccharose, le fructose, le maltose, le lactose ou le galactose.

10. Procédé selon l'une des revendications 1 à 9 caractérisé en ce que le microorganisme appartient au genre Xanthomonas.

11. Moûts de fermentation et poudres de polysaccharides obtenus par le procédé selon l'une des revendications 1 à 10.

12. Application des polysaccharides obtenus par le procédé selon l'une des revendications 1 à 10 à la récupération assistée du pétrole.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | FR-A-2 414 555 (RHONE-POULENC) <br> * Page 1; revendications; page 2, lignes 26-29 * <br> --- | 1,10-12 | C 12 P 19/06 <br> C 12 P 19/04 <br> E 21 B 43/25 // <br> (C 12 P 19/06 <br> C 12 R 1:64 ) |
| X | US-A-3 271 267 (R.O. WEBER) <br> * Colonne 5, lignes 46-49 * <br> --- | 1,10-12 | |
| D,X | US-A-3 433 708 (W.H. McNEELY) <br> * Colonne 4, ligne 53 - colonne 5, ligne 21 * <br> --- | 1,10-12 | |
| X | GB-A-1 416 013 (KELCO) <br> * Page 4, lignes 36-63 * <br> --- | 1,11,12 | |
| X | EP-A-0 112 661 (IMPERIAL BIOTECHNOLOGY) <br> * Exemple 2 * <br> ----- | 1,11 | |

**DOMAINES TECHNIQUES RECHERCHES (Int. Cl.5)**

C 12 P

Le présent rapport a été établi pour toutes les revendications

| Lieu de la recherche | Date d'achèvement de la recherche | Examinateur |
|---|---|---|
| LA HAYE | 08-11-1989 | SOMERVILLE F.M. |

EPO FORM 1503 03.82 (P0402)